# EUROPEAN PATENT SPECIFICATION

(11) **EP 1 117 798 B1**
(45) Date of publication and mention of the grant of the patent: **27.12.2006**
(21) Application number: 99945966.2
(22) Date of filing: 05.10.1999
(51) Int. Cl.: C12N 15/31, C12N 15/81, C07K 14/38

(54) **FUNGAL TRANSCRIPTIONAL ACTIVATOR PRTT FROM ASPERGILLUS NIGER, USEFUL IN METHODS FOR PRODUCING POLYPEPTIDES**
TRANSKRIPTIONSAKTIVATOR PRTT AUS DEM PILZ ASPERGILLUS NIGER VERWENDBAR IN VERFAHREN ZUR HERSTELLUNG VON POLYPEPTIDEN
ACTIVATEUR TRANSCRIPTIONNEL FONGIQUE PRTT D'ASPERGILLUS NIGER UTILISABLE DANS DES PROCEDES DE PRODUCTION DE POLYPEPTIDES

(30) Priority: 05.10.1998 DK 125898
(43) Date of publication of application: 25.07.2001
(73) Proprietor: Novozymes A/S, 2880 Bagsvaerd (DK)
(72) Inventor: HJORT, Carsten, DK-2880 Bagsvaerd (DK); VAN DEN HONDEL, Cees, A., M., J., J., NL-2804 PZ Gouda (NL); PUNT, Peter, J., NL-3994 XT Houten (NL); SCHUREN, Frank, H., J., NL-3906 ZK Veenendaal (NL)
(86) International application number: PCT/DK1999/000524
(87) International publication number: WO 2000/020596

(56) References cited:
- EP-A- 0 327 797
- WO-A-95/35385
- WO-A-97/12045
- WO-A-97/35956
- MATTERN I E ET AL.: "Isolation and characterization of mutants of Aspergillus niger deficient in extracellular proteases" MOLECULAR AND GENERAL GENETICS, vol. 234, no. 2, August 1992 (1992-08), pages 332-336, XP002127868 cited in the application
- VAN DEN HOMBERGH J P T W ET AL: "Aspergillus as a host for heterologous protein production: the problem of proteases" TRENDS IN BIOTECHNOLOGY,GB,ELSEVIER PUBLICATIONS, CAMBRIDGE, vol. 15, no. 7, July 1997 (1997-07), page 256-263 XP004081327 ISSN: 0167-7799
- VAN DEN HOMBERGH J P T W ET AL.: "Production of the homologous pectin lyase B protein in six genetically defined protease-deficient Aspergillus niger mutant strains" CURRENT GENETICS, vol. 32, no. 1, July 1997 (1997-07), pages 73-81, XP000867288

## Description

### Background of the Invention

### Field of the Invention

The present invention relates to isolated nucleic acid sequences encoding polypeptides having transcriptional activation activity and to the polypeptides. The invention also relates to nucleic acid constructs, vectors and host cells comprising the nucleic acid sequences. The invention further relates to methods for producing the polypeptides.

### Description of the Related Art

The use of recombinant host cells in the expression of heterologous proteins has in recent years greatly simplified the production of large quantities of commercially valuable proteins which otherwise are obtainable only by purification from their native sources. Currently, there is a varied selection of expression systems from which to choose for che production of any given protein, including eubacterial and eukaryotic hosts. The selection of an appropriate expression system often depends not only on the ability of the host cell to produce adequate yields of the protein in an active state, but, to a large extent, may also be governed by the intended end use of the protein.

One problem frequently encountered is the high level of proteolytic enzymes produced by a given host cell or present in the culture medium. One suggestion has been to provide host organisms deprived of the ability to produce specific proteolytic compounds. For example, WO 90/00192 (Genencor, Inc.) describes filamentous fungal hosts incapable of secreting enzymatically active aspartic proteinase. EP 574 347 (Ciba Geigy AG) describes *Aspergillus* hosts defective in a serine protease of the subtilisin-type. WO 98/12300 (Novo Nordisk A/S) describes hosts defective in a metalloprotease and an alkaline protease. WO 97/12045 (Genencor, Inc.) describes yeast and bacterial host systems which are rendered protease deficient resulting from a disruption of a promoter sequence involved in the regulation of a protease gene.

Mattern, I.E., et al., (1992. Mol Gen Genet 234:332-336) describe a mutant strain of *Aspergillus niger*, which was shown to have only 1 to 2% of the extracellular protease activity of the parent strain, apparently due to a deficiency of at least two proteases, aspergillopepsin A and aspergillopepsin B. It was suggested that the protease deficient phenotype could result from a regulatory mutation affecting the expression of the genes coding for both proteases.

The initiation of eukaryotic transcription at a specific promoter or set of promoters requires a eukaryotic transcriptional activator which is a polypeptide, but which is not itelf part of RNA polymerase. Many transcriptional activators bind to a specific site on the promoter to form a functional promoter necessary for the initiation of transcription of the polypeptide encoding sequence. However, a transcriptional activator may also be incorporated into an initiation complex only in the presence of other polypeptides. Polypeptides with transcriptional activation activity have been described in fungi, and a list of such polypeptides has been published (Dhawale, S.S., and Lane, A.C. 1993. Nucleic Acid Research 21:5537-5546).

### Solution proposed by the invention:

It is an object of the present invention to provide improved methods for increasing production of polypeptides in host cells in which the activity of a transcriptional activator involved in the regulation of protease production has been modified.

### Summary of the Invention

A first aspect of the present invention relates to an isolated nucleic acid sequence encoding a polypeptide having transcriptional activation activity on protease promoters, selected from the group consisting of:
(a) a nucleic acid sequence having at least 70% identity with the nucleic acid sequence of SEQ ID NO;1;
(b) a nucleic acid sequence encoding a polypeptide having an amino acid sequence which has at least 50% identity with the amino acid sequence of SEQ ID NO:2;
(c) a nucleic acid sequence which hybridizes under medium stringency conditions with (i) the nucleic acid sequence of SEQ ID NO:1, or (ii) its complementary strand, wherein the stringency conditions are defined by prehybridization and hybridization at 42°C in 5x SSPE, 0.3% SDS, 200 µg/ml sheared and denatured salmon sperm DNA, and 35% formamide, and wash conditions are defined by 50°C for 30 minutes in 2X SSC, 0.2% SDS; and
(d) a subsequence of (a), (b) or (c), wherein the subsequence encodes a polypeptide with the amino acid sequence of SEQ ID NO:3.

In another aspect, the invention also relates to nucleic acid constructs, vectors and host cells comprising the nucleic acid sequences, and to the polypeptides encoded by the nucleic acid sequences. The invention further relates to a method for producing the polypeptide.

### Brief Description of the Figures

Figure 1 shows a restriction map of the plasmid pPAP, the construction of which is described in Example 1.
Figure 2 shows a restriction map of the plasmid pAopyrGcosArpl, the construction of which is described in Example 1.
Figure 3 shows a restriction map of the plasmid pEES1, the construction of which is described in Example 1.
Figure 4 shows a restriction map of the plasmid pDprt, the construction of which is described in Example 3.
Figure 5 shows a restriction map of the plasmid pGPprt, the construction of which is described in Example 4.

### Detailed Description of the Invention

### Nucleic Acid Sequences Encoding Transcriptional Activators

A first aspect of the present invention relates to an isolated nucleic acid sequence encoding a polypeptide having transcriptional activation activity on protease promotors selected from the group consisting of:
(a) a nucleic acid sequence having at least 70% identity with the nucleic acid sequence of SEQ ID NO:1;
(b) a nucleic acid sequence encoding a polypeptide having an amino acid sequence which has at least 50% identity with the amino acid sequence of SEQ ID NO:2;
(c) a nucleic acid sequence which hybridizes under medium stringency conditions with (i) the nucleic acid sequence of SEQ ID NO:1, or (ii) its complementary strand, wherein the medium stringency conditions are defined by prehybridization and hybridization at 42°C in 5x SSPE, 0.3% SDS, 200 µg/ml sheared and denatured salmon sperm DNA, and 25% formamide, and wash conditions are defined by 50°C for 30 minutes in 2X SSC, 0.2% SDS; and
(d) a subsequence of (a), (b) or (c), wherein the subsequence encodes a polypeptide with the amino acid sequence of SEQ ID NO:3.
   The term "transcriptional activator" as used herein refers to a polypeptide which has the capability to activate a specific promoter or set of promoters necessary for the initiation of transcription of the polypeptide encoding sequence to which it is linked.

The term "isolated nucleic acid sequence" as used herein refers to a nucleic acid sequence which is essentially free of other nucleic acid sequences, *e.g.,* at least about 20% pure, preferably at least about 40% pure, more preferably at least about 60% pure, even more preferably at least about 80% pure, most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

In a preferred embodiment, the nucleic acid sequence has a degree of identity to the nucleic acid sequence set forth in SEQ ID NO:1 of at least about 70%, preferably at least about 80%, more preferably at least about 85%, even more preferably at least about 90%, most preferably at least about 95%, and even most preferably at least about 97% identity, which encodes an active polypeptide. For purposes of the present invention, the degree of identity between two nucleic acid sequences is determined by the Clustal method (Higgins, 1989, *CABIOS* 5:151-153) with an identity table, a gap penalty of 10, and a gap length penalty of 10.

In an even more preferred embodiment, the nucleic acid sequence encoding a transcriptional activator has a nucleic acid sequence as set forth in SEQ ID NO:1.

Modification of a nucleic acid sequence encoding a polypeptide of the present invention may be necessary for the synthesis of polypeptides substantially similar to the polypeptide. The term "substantially similar" to the polypeptide refers to non-naturally occurring forms of the polypeptide. These polypeptides may differ in some engineered way from the polypeptide isolated from its native source. For example, it may be of interest to synthesize variants of the polypeptide where the variants differ in specific activity, binding specificity and/or affinity, or the like using, *e.g*., site-directed mutagenesis. The analogous sequence may be constructed on the basis of the nucleic acid sequence presented as the polypeptide encoding part of SEQ ID NO:1, *e*.*g*., a subsequence thereof, and/or by introduction of nucleotide substitutions which do not give rise to another amino acid sequence of the polypeptide encoded by the nucleic acid sequence, but which corresponds to the codon usage of the host organism intended for production of the enzyme, or by introduction of nucleotide substitutions which may give rise to a different amino acid sequence. For a general description of nucleotide substitution, see, *e.g*., Ford et *al.,* 1991, *Protein*

### Expression and Purification 2: 95-107.

In another preferred embodiment, the present invention relates to isolated nucleic acid sequences encoding polypeptides having an amino acid sequence which has a degree of identity to the amino acid sequence set forth in SEQ ID NO:2 of at least about 50%, preferably at least about 60%, preferably at least about 70%, more preferably at least about 80%, even more preferably at least about 90%, most preferably at least about 95%, and even most preferably at least about 97%, which qualitatively retain the transcriptional activation activity of the polypeptides (hereinafter "homologous polypeptides").

In a preferred embodiment, the homologous polypeptides have an amino acid sequence which differs by five amino acids, preferably by four amino acids, more preferably by three amino acids, even more preferably by two amino acids, and most preferably by one amino acid from the amino acid sequence set forth in SEQ ID NO:2. For purposes of the present invention, the degree of identity between two amino acid sequences is determined by the Clustal method (Higgins, 1989, *supra*) with an identity table, a gap penalty of 10, and a gap length penalty of 10.

Hybridization indicates that by methods of standard Southern blotting procedures, the nucleic acid sequence hybridizes to an oligonucleotide probe corresponding to the polypeptide encoding part of the nucleic acid sequence shown in SEQ ID NO:1, under medium to high stringency conditions (*i.e.,* prehybridization and hybridization at 42°C in 5x SSPE, 0.3% SDS, 200 µg/ml sheared and denatured salmon sperm DNA, and either 35 or 50% formamide for medium and high stringencies, respectively). In order to identify a clone or DNA which is homologous with SEQ ID NO:1, the hybridization reaction is washed three times for 30 minutes each using 2X SSC, 0.2% 5DS preferably at least 50°C, more preferably at least 55°C, more preferably at least 60°C, more preferably at least 65°C, even more preferably at least 70°C, and most preferably at least 75°C.

The nucleic acid sequence of SEQ ID NO:1, or a subsequence thereof, as well as the amino acid sequence of SEQ ID NO:2, or a partial sequence thereof, or the amino acid sequence of SEQ ID NO:3, may be used to design an oligonucleotide probe to identify and isolate or clone a homologous gene of any genus or species according to methods well known in the art.

In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 40 nucleotides in length. Longer probes can also be used. Both DNA and RNA probes can be used. The probes are typically labeled for detecting the corresponding gene (for example, with ³²P, ³H, ³⁵S, biotin, or avidin). For example, molecules to which a ³²P-, ³H- or ³⁵S-labelled oligonucleotide probe hybridizes may be detected by use of X-ray film.

Thus, a genomic, cDNA or combinatorial chemical library prepared from such other organisms may be screened for DNA which hybridizes with the probes described above and which encodes a polypeptide with transcriptional activation activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. A clone or DNA which is homologous to SEQ ID NO:1 may then be identified following standard Southern blotting procedures.

An allelic variant denotes any of two or more alternative forms of a gene occupying the same chomosomal locus. Allelic variation arises naturally through mutation, and may result in phenotypic polymorphism within populations. Gene mutations can be silent (*i.e*., no change in the encoded polypeptide) or may encode polypeptides having altered amino acid sequences.

The term "allelic variant of a polypeptide" is a polypeptide encoded by an allelic variant of a gene. In a preferred embodiment, the nucleic acid sequence encoding a transcriptional activator of the present invention is an allelic variant of a nucleic acid sequence selected from the group consisting of nucleic acid sequences encoding a polypeptide comprising the amino acid sequence of SEQ ID NO:3.

The present invention also encompasses nucleic acid sequences which differ from SEQ ID NO:1 by virtue of the degeneracy of the genetic code. The present invention also relates to subsequences of SEQ ID NO:1. In a preferred embodiment, a subsequence of SEQ ID NO:1 encodes the polypeptide sequence shown in SEQ ID NO:3.

The techniques used to isolate or clone a nucleic acid sequence encoding a polypeptide are known in the art and include isolation from genomic DNA, preparation from cDNA, or a combination thereof. The cloning of the nucleic acid sequences of the present invention from such genomic DNA can be effected, *e.g*., by using methods based on polymerase chain reaction (PCR) or antibody screening of expression libraries to detect cloned DNA fragments with shared structural features. (See, *e.g*., Innis et *al.,* 1990, *PCR: A Guide to Methods and Application,* Academic Press, New York.) Other nucleic acid amplification procedures such as ligase chain reaction (LCR), ligated activated transcription (LAT) and nucleic acid sequence-based amplification (NASBA) may be used. The nucleic acid sequence may be cloned from a microorganism, or another or related organism and thus, for example, may be an allelic or species variant of the polypeptide encoding region of the nucleic acid sequence.

The transcriptional activators encoded by nucleic acid sequences which hybridize with an oligonucleotide probe which hybridizes with the nucleic acid sequence of SEQ ID NO:1 or its complementary strand may be obtained from microorganisms of any genus.

In a preferred embodiment, the transcriptional activators may be obtained from a fungal cell. "Fungi" as used herein includes the phyla Ascomycota, Basidiomycota, Chytridiomycota, and Zygomycota (as defined by Hawksworth, et *al.,* in *Ainsworth and Bisby's Dictionary of The Fungi,* 8th edition, 1995, CAB International, University Press, Cambridge, UK) as well as the Oomycota (as cited in Hawksworth *et al.,* 1995, *supra,* page 171) and all mitosporic fungi (Hawksworth *et al.,* 1995, *supra*)*.*

In preferred embodiment, the fungal source is a filamentous fungal strain. "Filamentous fungi" include all filamentous forms of the subdivision Eumycota and Oomycota (as defined by Hawksworth *et al.,* 1995, *supra*). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. Filamentous fungal strains include, but are not limited to, strains of *Acremonium*, *Aspergillus, Aureobasidium, Cryptococcus, Filibasidium, Fusarium, Humicola, Magnaporthe, Mucor*, *Myceliophthora, Neocallimastix, Neurospora, Paecilomyces, Penicillium, Piromyces, Schizophyllum, Talaromyces, Thermoascus, Thielavia, Tolypocladium,* and *Trichoderma.*

In a more preferred embodiment, the nucleic acid sequence encoding a transcriptional activator of the present invention is obtained from a strain of *Aspergillus*, such as *A*. *awamori* or *A. nidulans*. Preferably, the nucleic acid sequence is obtained from a strain of *A. niger* or *A. oryzae.* Even more preferably, the nucleic acid sequence is obtained from an isolate of a strain of *A*. *niger,* DSM 12298; e.g., the nucleic acid sequence set forth in SEQ ID NO:1.

In another more preferred embodiment, the nucleic acid sequence encoding a transcriptional activator of the present invention is obtained from a strain of *Fusarium*, such as *F*. *oxysporum.* Preferably, the strain is a strain of *F*. *venenatum* (Nirenberg sp. nov.).

In another preferred embodiment, the nucleic acid sequence encoding a transcriptional activator of the present invention is obtained from a yeast strain, such as a *Candida, Kluyveromyces, Schizosaccharomyces,* or *Yarrowia* strain. Preferably, the strain is a strain of *Hansenula, Pichia,* or *Saccharomyces.*

It will be understood that for the aforementioned species, the invention encompasses both the perfect and imperfect states, and other taxonomic equivalents, e.g., anamorphs, regardless of the species name by which they are known. Those skilled in the art will readily recognize the identity of appropriate equivalents. For example, the polypeptides may be obtained from microorganisms which are taxonomic equivalents of *Aspergillus* as defined by Raper, K.D. and Fennel, D.I. (1965. *The Genus Aspergillus,* The Wilkins Company, Baltimore MD). regardless of the species name by which they are known. *Aspergilli* are mitosporic fungi characterized by an aspergillum comprised of a conidiospore stipe with no known teleomorphic states terminating in a vesicle, which in turn bears one or two layers of synchronously formed specialized cells, variously referred to as sterigmata or phialides, and asexually formed spores referred to as conidia. Known teleomorphs of *Aspergillus* include *Eurotium, Neosartorya,* and *Emericella.* Strains of *Aspergillus* and teleomorphs thereof are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

Furthermore, such transcriptional activators may be identified and obtained from other sources including microorganisms isolated from nature (e.g., soil, composts, water, etc.) using the above-mentioned probes. Techniques for isolating microorganisms from natural habitats are well known in the art. The nucleic acid sequence may then be derived by similarly screening a genomic or cDNA library of another microorganism. Once a nucleic acid sequence encoding a transcriptional activator has been detected with the probe(s), the sequence may be isolated or cloned by utilizing techniques which are known to those of ordinary skill in the art (see, *e.g.,* J. Sambrook, E.F. Fritsch, and T. Maniatus, 1989, *Molecular Cloning, A Laboratory Manual,* 2d edition, Cold Spring Harbor, New York).

In another preferred embodiment, the isolated nucleic acid sequence encodes a polypeptide comprising the amino acid sequence of SEQ ID NO:2, or a fragment thereof, which has transcriptional activation activity.

In another preferred embodiment, the isolated nucleic acid sequence encodes a polypeptide comprising the amino acid sequence of SEQ ID NO:3.

The present invention also relates to isolated nucleic acid sequences encoding a transcriptional activator of the present invention, which, *e.g.,* using methods of standard Southern blotting procedures described above (*cf*., Sambrook, et *al.,* 1989, *supra*), hybridize under medium stringency conditions, and more preferably high stringency conditions, with an oligonucleotide probe which hybridizes under the same conditions with the nucleic acid sequence set forth in SEQ ID NO:1 or its complementary strand.

In another more preferred embodiment, the nucleic acid sequence is the nucleic acid sequence encoding a polypeptide which has DNA binding activity contained in the plasmid pEES which is contained in *Escherichia coli* DSM 12294.

### Nucleic Acid Constructs

Another aspect of the present invention relates to nucleic acid constructs comprising a nucleic acid sequence encoding a transcriptional activator of the present invention operably linked to one or more control sequences which direct the production of the transcriptional activator in a suitable expression host. In a preferred embodiment, the nucleic acid sequence encodes a polypeptide which is contained in the plasmid pEES harboured in *Escherichia coli* DSM 12294.

Expression will be understood to include any step involved in the production of the polypeptide including, but not limited to, transcription, post-transcriptional modification, translation, post-translational modification, and secretion. Manipulation of the nucleic acid sequence encoding a polypeptide prior to its insertion into a vector may be desirable or necessary depending on the expression vector. The techniques for modifying nucleic acid sequences utilizing cloning methods are well known in the art.

"Nucleic acid construct" is defined herein as a nucleic acid molecule, either single- or double-stranded, which is isolated from a naturally occurring gene or which has been modified to contain segments of nucleic acid which are combined and juxtaposed in a manner which would not otherwise exist in nature. The term nucleic acid construct is synonymous with the term expression cassette when the nucleic acid construct contains all the control sequences required for expression of a coding sequence. The term "coding sequence" as defined herein is a sequence which is transcribed into mRNA and translated into a transcriptional avtivator of the present invention. The boundaries of the coding sequence are generally determined by the ATG start codon at the 5' end of the mRNA and a transcription terminator sequence located just downstream of the open reading frame at the 3' end of the mRNA. A coding sequence can include, but is not limited to, DNA, cDNA, and recombinant nucleic acid sequences.

The term "control sequences" is defined herein to include all components which are necessary or advantageous for the expression of a polypeptide. Each control sequence may be native or foreign to the nucleic acid sequence encoding the polypeptide. Such control sequences include, but are not limited to, a leader, a polyadenylation sequence, a propeptide sequence, a promoter, a signal sequence, and a transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a polypeptide. The term "operably linked" is defined herein as a configuration in which a control sequence is appropriately placed at a position relative to the coding sequence of the DNA sequence such that the control sequence directs the production of a polypeptide.

The control sequence may be an appropriate promoter sequence, a nucleic acid sequence which is recognized by a host cell for expression of the nucleic acid sequence. The promoter sequence contains transcriptional control sequences which mediate the expression of the polypeptide. The promoter may be any nucleic acid sequence which shows transcriptional activity in the cell including mutant, truncated, and hybrid promoters, and may be obtained from genes encoding extracellular or intracellular polypeptides either homologous or heterologous to the cell.

The control sequence may also be a suitable transcription terminator sequence, a sequence recognized by a filamentous fungal cell to terminate transcription. The terminator sequence is operably linked to the 3' terminus of the nucleic acid sequence encoding the polypeptide. Any terminator which is functional in the cell may be used in the present invention.

Preferred terminators for filamentous fungal cells are obtained from the genes encoding *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

The control sequence may also be a suitable leader sequence, a nontranslated region of a mRNA which is important for translation by the filamentous fungal cell. The leader sequence is operably linked to the 5' terminus of the nucleic acid sequence encoding the polypeptide. Any leader sequence which is functional in the cell may be used in the present invention.

Preferred leaders for filamentous fungal cells are obtained from the genes encoding *Aspergillus oryzae* TAKA amylase and *Aspergillus nidulans* triose phosphate isomerase.

The control sequence may also be a polyadenylation sequence, a sequence which is operably linked to the 3' terminus of the nucleic acid sequence and which, when transcribed, is recognized by the filamentous fungal cell as a signal to add polyadenosine residues to transcribed mRNA. Any polyadenylation sequence which is functional in the cell may be used in the present invention.

Preferred polyadenylation sequences for filamentous fungal cells are obtained from the genes encoding *Aspergillus oryzae* TAKA amylase, *Aspergillus niger* glucoamylase, *Aspergillus nidulans* anthranilate synthase, and *Aspergillus niger* alpha-glucosidase.

The control sequence may also be a signal peptide coding region, which codes for an amino acid sequence linked to the amino terminus of the polypeptide which can direct the encoded polypeptide into the cell's secretory pathway. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not normally contain a signal peptide coding region. Alternatively, the foreign signal peptide coding region may simply replace the natural signal peptide coding region in order to obtain enhanced secretion of the polypeptide. The signal peptide coding region may be obtained from a glucoamylase or an amylase gene from an *Aspergillus* species, or a lipase or proteinase gene from a *Rhizomucor* species. However, any signal peptide coding region which directs the expressed polypeptide into the secretory pathway of a filamentous fungal cell may be used in the present invention.

An effective signal peptide coding region for filamentous fungal cells is the signal peptide coding region obtained from the *Aspergillus oryzae* TAKA amylase gene, *Aspergillus niger* neutral amylase gene, *Rhizomucor* miehei aspartic proteinase gene, or *Humicola lanuginosa* cellulase gene.

The control sequence may also be a propeptide coding region, which codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the *Rhizomucor miehei* aspartic proteinase gene, or the *Myceliophthora thermophila* laccase gene (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

### Expression Vectors

The present invention also relates to recombinant expression vectors comprising a nucleic acid sequence of the present invention, a promoter, and transcriptional and translational stop signals. The various nucleic acid and control sequences described above may be joined together to produce a recombinant expression vector which may include one or more convenient restriction sites to allow for insertion or substitution of the nucleic acid sequence encoding the polypeptide at such sites. Alternatively, the nucleic acid sequence encoding the polypeptide may be expressed by inserting the sequence or a nucleic acid construct comprising the sequence into an appropriate vector for expression. In creating the expression vector, the coding sequence is located in the vector so that the coding sequence is operably linked with the appropriate control sequences for expression, and possibly secretion.

The recombinant expression vector may be any vector (*e.g*., a plasmid or virus) which can be conveniently subjected to recombinant DNA procedures and can bring about the expression of the nucleic acid sequence encoding the polypeptide. The choice of the vector will typically depend on the compatibility of the vector with the filamentous fungal cell into which the vector is to be introduced. The vectors may be linear or closed circular plasmids. The vector may be an autonomously replicating vector, *i.e*., a vector which exists as an extrachromosomal entity, the replication of which is independent of chromosomal replication, *e.g*., a plasmid, an extrachromosomal element, a minichromosome, or an artificial chromosome. The vector may contain any means for assuring self-replication. Alternatively, the vector may be one which, when introduced into the filamentous fungal cell, is integrated into the genome and replicated together with the chromosome(s) into which it has been integrated. The vector system may be a single vector or plasmid or two or more vectors or plasmids which together contain the total DNA to be introduced into the genome of the filamentous fungal cell, or a transposon.

The vectors preferably contain one or more selectable markers which permit easy selection of transformed cells. A selectable marker is a gene the product of which provides for biocide or viral resistance, resistance to heavy metals, prototrophy to auxotrophs, and the like. A selectable marker for use in a filamentous fungal cell may be selected from the group including, but not limited to, *amdS* (acetamidase), *argB* (ornithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents from other species. Preferred for use in an *Aspergillus* cell are the *amdS* and *pyrG* genes of *Aspergillus nidulans* or *Aspergillus oryzae* and the bar gene of *Streptomyces hygroscopicus.*

The vectors preferably contain an element(s) that permits stable integration of the vector into the host cell genome or autonomous replication of the vector in the cell independent of the genome of the cell.

### Host Cells

Another aspect of the present invention relates to host cells comprising a nucleic acid construct or an expression vector of the present invention.

The choice of a host cell in the methods of the present invention will to a large extent depend upon the source of the nucleic acid sequence encoding the polypeptide of interest.

The introduction of an expression vector or a nucleic acid construct into a filamentous fungal cell may involve a process consisting of protoplast formation, transformation of the protoplasts, and regeneration of the cell wall in a manner known *per se*. Suitable procedures for transformation of *Aspergillus* cells are described in EP 238 023 and Yelton et *al*., 1984, *Proceedings of the National Academy of Sciences USA* 81: 1470-1474. A suitable method of transforming *Fusarium* species is described by Malardier *et al.,* 1989, *Gene* 78: 147-156 or in WO 96/00787.

"Introduction" means introducing a vector comprising the nucleic acid sequence encoding the polypeptide into a filamentous fungal cell so that the vector is maintained as a chromosomal integrant or as a self-replicating extrachromosomal vector. Integration is generally considered to be an advantage as the nucleic acid sequence is more likely to be stably maintained in the cell. Integration of the vector into the chromosome occurs by homologous recombination, nonhomologous recombination, or transposition.

For integration into the host cell genome, the vector may rely on the nucleic acid sequence encoding the polypeptide or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. These nucleic acid sequences may be any sequence that is homologous with a target sequence in the genome of the host cell, and, furthermore, may be non-encoding or encoding sequences.

For autonomous replication, the vector may further comprise an origin of replication enabling the vector to replicate autonomously in the host cell.

The procedures used to ligate the elements described above to construct the recombinant expression vectors are well known to one skilled in the art (see, *e.g.*, Sambrook, *et al., supra*).

In a preferred embodiment, the filamentous fungal host cell is a cell of a species of, but not limited to, Acremonium, *Aspergillus, Fusarium, Humicola, Mucor, Myceliophthora, Neurospora, Penicillium, Thielavia*, *Tolypocladium,* or *Trichoderma.*

In a more preferred embodiment, the filamentous fungal cell is an *Aspergillus* cell. In another more preferred embodiment, the filamentous fungal cell is an *Acremonium* cell. In another more preferred embodiment, the filamentous fungal cell is a *Fusarium* cell. In another more preferred embodiment, the filamentous fungal cell is a *Humicola* cell. In another more preferred embodiment, the filamentous fungal cell is a *Mucor* cell. In another more preferred embodiment, the filamentous fungal cell is a *Myceliophthora* cell. In another more preferred embodiment, the filamentous fungal cell is a *Neurospora* cell. In another more preferred embodiment, the filamentous fungal cell is a *Penicillium* cell. In another more preferred embodiment, the filamentous fungal cell is a *Thielavia* cell. In another more preferred embodiment, the filamentous fungal cell is a *Tolypocladium* cell. In another more preferred embodiment, the filamentous fungal cell is a *Trichoderma* cell.

In a most preferred embodiment, the filamentous fungal cell is an *Aspergillus awamori, Aspergillus foetidus, Aspergillus japonicus*, *Aspergillus nidulans, Aspergillus niger* or *Aspergillus oryzae* cell. In another most preferred embodiment, the filamentous fungal cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sulphureum, Fusarium toruloseum, Fusarium trichothecioides,* or *Fusarium venenatum* cell. In an even most preferred embodiment, the filamentous fungal cell is a *Fusarium venenatum* (Nirenberg sp. nov.). In another most preferred embodiment, the filamentous fungal cell is a *Humicola insolens* or *Humicola lanuginosa* cell. In another most preferred embodiment, the filamentous fungal cell is a *Mucor miehei* cell. In another most preferred embodiment, the filamentous fungal cell is a *Myceliophthora thermophilum* cell. In another most preferred embodiment, the filamentous fungal cell is a *Neurospora crassa* cell. In another most preferred embodiment, the filamentous fungal cell is a *Penicillium purpurogenum* cell. In another most preferred embodiment, the filamentous fungal cell is a *Thielavia terrestris* cell. In another most preferred embodiment, the *Trichoderma* cell is a *Trichoderma harzianum, Trichoderma koningii, Trichoderma longibrachiatum, Trichoderma* reesei or *Trichoderma viride* cell.

### Polypeptides having Transcriptional Activation Activity

Another aspect of the present invention relates to an isolated polypeptide selected from the group consisting of:
(a) a polypeptide which is encoded in a nucleic acid sequence which hybridizes under medium stringency conditions with (i) the nucleic acid sequence of SEQ ID NO:1; (ii) its complementary strand, or (iii) a subsequence of SEQ ID NO:1 which encodes a polypeptide fragment which has transcriptional activation activity, wherein the medium stringency conditions are defined by prehybridization and hybridization at 42°C in 5X SSPE, 0.3% SDS, 200 µg/ml sheared and denatured salmon sperm DNA, and 35% formamide, and wash conditions are defined at 50°C for 30 minutes in 2X SSC, 0.2% SDS;
(b) a polypeptide having an amino acid sequence which has at least 50% identity with the amino acid sequence of SEQ ID NO:2; and
(c) a polypeptide comprising the amino acid sequence of SEQ ID NO:3.

The transcriptional activator may be isolated using techniques as described herein. As defined herein, an "isolated" polypeptide is a polypeptide which is essentially free of other polypeptides, *e*.*g*., at least about 20% pure, preferably at least about 40% pure, more preferably about 60% pure, even more preferably about 80% pure, most preferably about 90% pure, and even most preferably about 95% pure, as determined by SDS-PAGE.

The present invention also relates to isolated polypeptides having an amino acid sequence which has a degree of identity to the amino acid sequence of SEQ ID NO:2 of at least about 50%, preferably at least about 55%, preferably at least about 60%, preferably at least about 65%, preferably at least about 70%, preferably at least about 75%, preferably at least about 80%, more preferably at least about 85%, even more preferably at least about 90%, most preferably at least about 95%, and even most preferably at least about 97%, which have transcriptional activation activity.

In more preferred embodiment, the transcriptional activator of the present invention comprises the amino acid sequence of SEQ ID NO:2, or a fragment thereof, wherein the fragment retains transcriptional activation activity. In a most preferred embodiment, the polypeptide has the amino acid sequence of SEQ ID NO:2. A fragment of SEQ ID NO:2 is a polypeptide having one or more amino acids deleted from the amino and/or carboxy terminus of this amino acid sequence. Preferably, a fragment of SEQ ID NO:2 contains at least the polypeptide sequence shown in SEQ ID NO:3.

The amino acid sequences of the homologous polypeptides may differ from the amino acid sequence of SEQ ID NO:2 or SEQ ID NO:3 by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (such as arginine, lysine and histidine), acidic amino acids (such as glutamic acid and aspartic acid), polar amino acids (such as glutamine and asparagine), hydrophobic amino acids (such as leucine, isoleucine and valine), aromatic amino acids (such as phenylalanine, tryptophan and tyrosine), and small amino acids (such as glycine, alanine, serine, threonine and methionine). Amino acid substitutions which do not generally alter the specific activity are known in the art and are described, for example, by H. Neurath and R.L. Hill (1979. *The Proteins,* Academic Press, New York). The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

Also described is a transcriptional activator obtained from an *Aspergillus niger* strain, more preferably from *Aspergillus niger* AB4.1 (van Haringsveldt, W., et al., 1987. Mol. Gen. Genet. 206:71-75), and most preferably from *Aspergillus niger* 13PAP2, which has been deposited at DSM as DSM 12298, or a mutant strain thereof, harbouring, e.g., the polypeptide with the amino acid sequence of SEQ ID NO:2 or SEQ ID NO:3.

Also described is the transcriptional activator being the polypeptide encoded in the nucleic acid sequence contained in plasmid pEES which is contained in *Escherichia coli* DSM 12294.

The present invention further relates to methods for producing the transcriptional activator of the present invention comprising (a) cultivating a host cell harbouring a nucleic acid construct or an expression vector comprising a nucleic acid sequence encoding the transcriptional activator of the invention under conditions conducive for production of the polypeptide; and (b) recovering the polypeptide.

The present invention is further described by the following examples which should not be construed as limiting the scope of the invention.

### EXAMPLES

### Materials

Chemicals used as buffers and substrates were commercial products of at least reagent grade.

### Strains

AB4.1: a strain of *Aspergillus niger* which is a *cspA1 pyrG1* derivative of strain ATTC 9029 (van Hartingsveldt, W., et al., 1987. Mol. Gen. Genet. 206:71-75; Bos, C.J., et al., Curr. Genet. 14:437-443)

AB1.13: a protease deficient strain of *Aspergillus niger* derived from UV mutagenesis of AB4.1 (Mattern, I.E., et al., 1992. Mol. Gen. Genet. 234:332-336)

13PAP2: an AB1.13 derivative containing multiple copies of the *A*. *nidulans amdS* gene (Corrick, R. A., et al, 1987. Gene 53: 63 - 71) under control of the *pepA* promoter of *A*. *niger* (Jarai G. and Buxton F. 1994. Curr Genet 26:238-244). The strain has a protease deficient phenotype and is unable to grow on medium containing acetamide as the sole nitrogen source. Strain 13PAP2 has been deposited at DSM under the name DSM No. 12298.

4PAP6: an AB4.1 derivative containing multiple copies of the of *A. nidulans amdS* gene under control of the *pepA* promoter of *A. niger.* The strain does not have a protease deficient phenotype and is able to grow on medium containing acetamide as the sole nitrogen source.

N402: a strain of *Aspergillus niger,* deposited at the ATCC (Manassas VA, USA) as ATCC Number: 64974

MC1046: a strain of *E*. *coli,* deposited at the ATCC as ATCC Number: 35467

### Plasmids

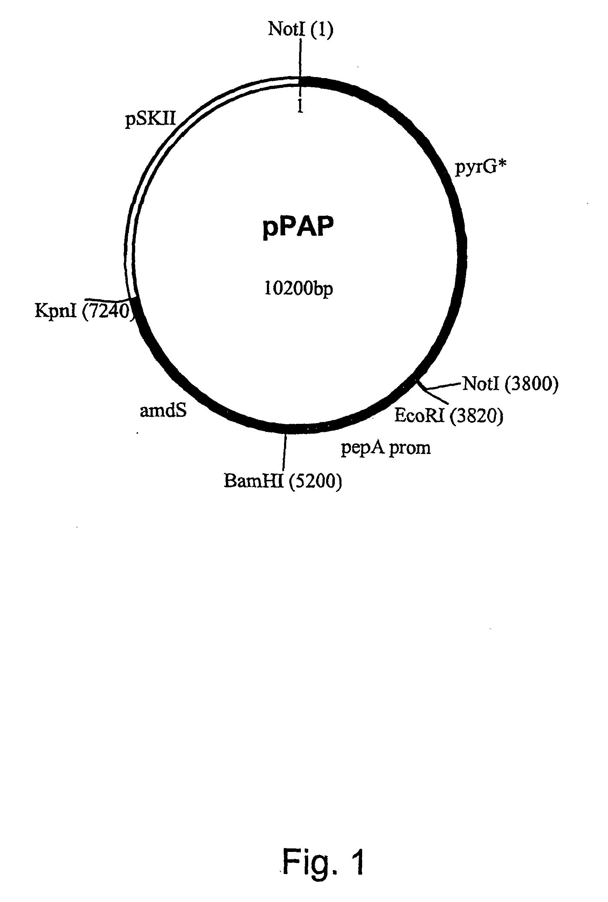

pPAP: constructed as described below in Example 1 and shown in Figure 1

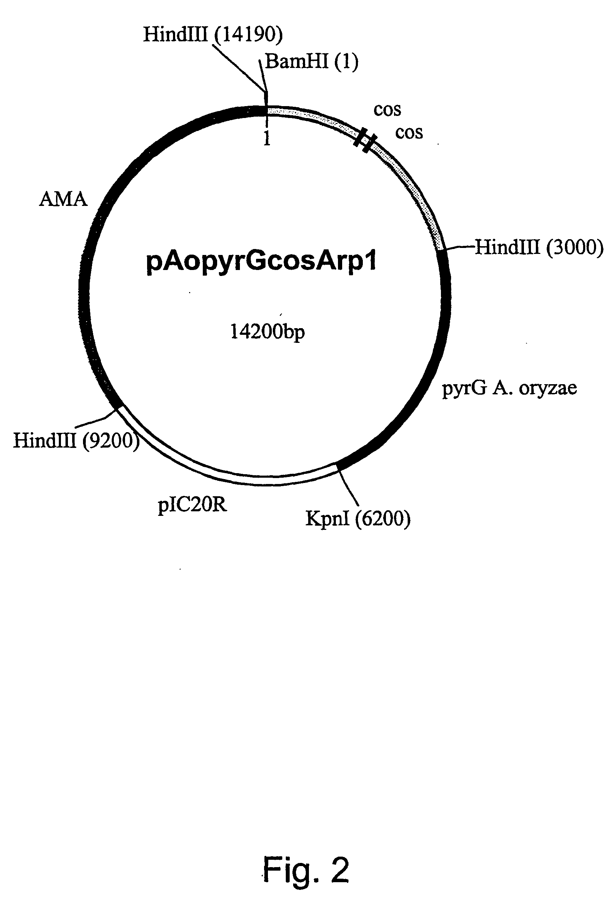

pAopyrGcosArp1: constructed as described below in Example 1 and shown in Figure 2

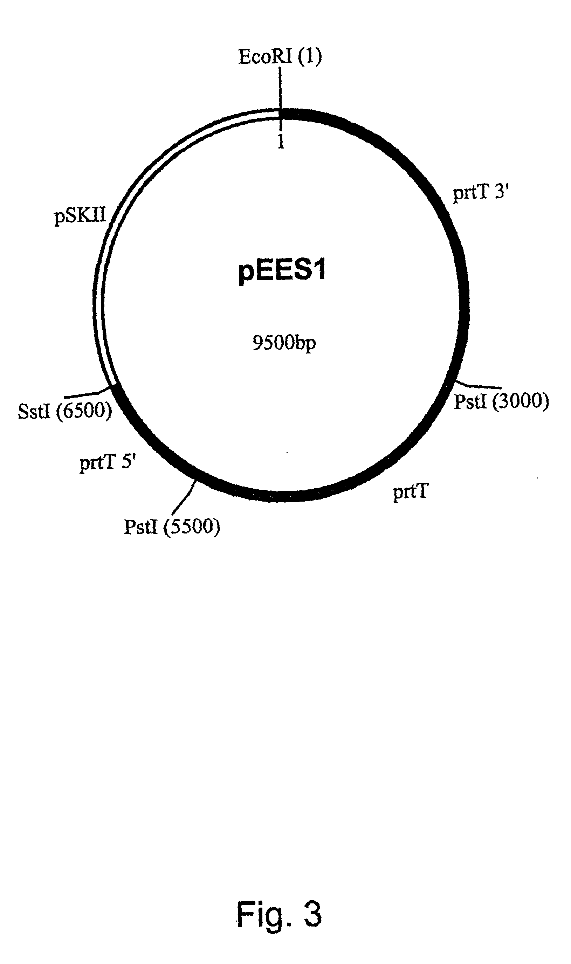

pEES1: constructed as described below in Example 1 and shown in Figure 3

p3SR2: contains the *A*. *nidulans amdS* gene as described by C.M. Corrick, A.P. Twomey, and M.J. Hynes (1987. Gene 53: 63-71)

pABPYRG*-Not: contains an inactivated *pyrG* gene as described by Verdoes, J.C., et al. (1994. Gene 145: 179-187)

pHelp1: contains the *pyrG* gene from *A*. *oryzae* as a selective marker and the AMA1 sequences which enable autonomous replication in *A. niger,* cloned into the *E. coli* vector pIC20R, as described by Gems, D., et al. (1991. Gene 98: 61-67)

pAnscos1: contains two cos sites as described by Osiewacz, H.D. (1994. Curr. Genet. 26: 87-90)

pA04-2: contains the *A*. *oryzae pyrG* gene as described by De Ruiter-Jacobs, Y.M.J.T., *et al.* (1989. Curr. Genet. 16: 159-163)

pAO4-13: contains the *A*. *oryzae pyrG* gene as described by De Ruiter-Jacobs, Y.M.J.T., *et al.* (1989. Curr. Genet. 16:159-163)
pUC19: as described by Yanisch-Perron, C., Vieira, J. and Messig, J. (1985, Gene 33:103-119)

### Example 1: Cloning of the prtT transcriptional activator from A. niger

*prtT* was cloned from 13PAP2, an *A*. *niger* mutant strain which is unable to express the *amdS* gene regulated by the *pepA* protease gene promoter and has a protease deficient phenotype (prt ) .

### Construction of the A. niger 13PAP2 reporter strain

The plasmid pPA1 was contructed by ligation of the following three fragments:
1) the *E*. *coli* vector pBlueScript II SK (Stratagene Cloning Systems, La Jolla CA, USA) digested with EcoRI and KpnI;
2) a 1.4 kb EcoRI/BamHI restriction fragment containing the 1.2 kb promoter region of the *pepA* gene linked to about 130 bp of the *amdS* coding sequence from the start codon to an internal BamHI site, amplified by PCR; and
3) a 2.1 kb BamHI/KpnI fragment from p3SR2 which contains most of the *A. nidulans amds* gene

Fragment 2 was constructed in two steps. In a first step genomic DNA from *A*. *niger* N402 prepared from protoplasts as described below in the section "Construction of the Cosmid Library" was used as the template, and the two oligonucleotides shown below, pepApr and pepA/amdS, were used as primers:
PepApr: CGG AAT TCG CAT GCT GGA GGT GCT TCT AA
pepA/amdS: TTC CCA GGA TTG AGG CAT TTT GAC CAC GAG AAT

The 1200 bp PCR product obtained from this reaction was then used as a primer in a second PCR reaction together with the oligonucleotide MBL1213 shown below, and plasmid p3SR2 as the template.
MBL1213: TAA CTT CCA CCG AGG TC

The product obtained by ligation of the three fragments described above was subsequently transfected into *E*. *coli* DH5α.

In the final construction procedure, pPA1 was digested with NotI and ligated to a 3.8 kb NotI fragment from pABPYRG*-Not, resulting in plasmid pPAP which is shown in Fig. 1. pPAP was transformed into *A*. *niger* AB 1.13, and a transformant with pPAP integrated into the *pyrG* locus in multicopy was isolated. A spontanous 5 flourotic acid (FOA) resistant, uridine-requiring mutant of this transformant that could be complemented with the *pyrG* gene was named 13PAP2.

### Construction of pAopyrGcosArp1

The plasmid pAopyrGcosArp1 was constructed by ligation and subsequent transfection into E. *coli* DH5a of the following three fragments:
1) the *E. coli* vector pHelp1 cut with Acc65I and BamHI
2) a 3.0 kb BamHI/HindIII fragment from pAnscos1 containing two cos sites
3) a 3.2 kb Acc65I/HindIII fragment from pAO4-2 containing the *A. oryzae pyrG* gene
   The resulting plasmid, pAopyrGcosArp1, is self-replicating in *Aspergilli* and can be selected for by growth on medium lacking uridine. pAopyrGcosArp1 is depicted in Fig. 2.

### Construction of the cosmid library

A cosmid library of *Aspergillus niger* was constructed using the "SuperCos1 cosmid vector kit" (Stratagene Cloning Systems, La Jolla CA, USA) according to the supplier's instructions.

Genomic DNA from *A*. *niger* N402 was prepared from protoplasts made by standard procedures.

After isolation the protoplasts were pelleted by centrifugation at 2000 rpm for 10 minutes in a Beckman GS-6R; the pellet was then suspended in a buffer containing 22.5 mM tri-isonaphtalene sulphonic acid, 275 mM para-aminosalicylic acid, 0.2 M Tris-HCl (pH 8.5), 0.25 M NaCl and 50 mM EDTA immediately followed by addition of 1 volume of phenol/chloroform (1:1). After careful mixing and centrifugation at 3000 rpm for 20 minutes the aqueous phase was decanted and DNA was precipitated using standard procedures.

The size of the genomic DNA was analysed by electrophoresis on a 0.3% agarose gel run for 20 hours at 30 volts at 4°C. The ethidium bromide stained gel showed that the recovered DNA ranged in size from 50 to greater than 100 kb. The DNA was partially digested using MboI. The size of the digested DNA was 30 to 50 kb as determined by the same type of gel analysis as above. The pAopyrGcosArp1 vector, purified using a kit from QIAGEN (Venlo, The Netherlands) following the manufacturer's instructions, was digested with BamHI, dephosphorylated and gel purified. Ligation and packaging were performed following standard procedures.

After titration of the library, all of the packaging mix from a single ligation and packaging was transfected into the host cell, MC1046, and plated on 50 µg/ml ampicillin LB plates. Approximately 40,000 colonies were obtained. Cosmid preparations from 10 colonies showed that they all had inserts of the expected size. The 40,000 colonies were then soaked in LB medium and scraped off of the plates, then aliquoted for storage in 15% glycerol at -80°C. This represents an approximate 40-fold amplification of the *A. niger* genome.

### Selection of prtT clones

Cosmid DNA was prepared from the library and introduced into 13PAP2 according to the transformation procedure described by P.J. Punt and C.A.M.J.J. Van Den Hondel (1992. *Methods Enzymol* 216: 447-457). Repeated efforts to select for the *pyrG* marker only resulted in a recovery of between 4000 to 30,000 transformants. A double selection for the *pyrG* marker and growth on medium containing acetamide as the sole nitrogen source resulted in a total of 65 primary transformants from five different experiments.

Each primary transformant was screened for protease activity, growth on medium containing acetamide as the sole nitrogen source and instability of the these two characteristics. An acetamidase⁺ phenotype, screened by growth on medium containing acetamide, is an indication of acetamidase activity resulting from activation of the *pepA* promoter in the reporter cassette in which the *pepA* promoter is linked to the *amdS* coding sequence. A protease⁺ phenotype was screened using minimal medium plates containing dialyzed skim milk as the sole nitrogen source (Mattern, I.E., et al., 1992. Mol Gen Genet 234:332-336). On these plates the wild-type AB4.1 strain makes a clear halo whereas the AB1.13 mutant produces a very small halo. This difference is not due to differences in the activity of *pepA* since a *pepA* deleted strain can also produce a large halo on these plates. Therefore, a large halo on milk plates indicates activation of other extracellular proteases.

Instability was tested by growing diluted spore stocks on medium containing uridine. Single-spore-derived colonies were picked from these plates and tested for protease activity and growth on acetamide. The screening results revealed that in more than 70% of the colonies both characteristics were lost. Therefore, the two phenotypes were either lost or retained together, indicating that activation of the *pepA* promoter and other protease promoters is coordinately regulated and linked to the presence of the *pyrG* marker. The gene responsible for this phenotype was named *prtT.* Twelve acetamidase⁺, protease⁺ transformants were then isolated.

### Isolation of the prtT gene

In order to rescue the *prtT* gene from the acetamidase⁺, protease⁺ transformants of 13PAP2, DNA was prepared from mycelium grown in minimal medium as previously described. This DNA was used in an attempt to transform competent E. *coli* DH5a cells. Several hundreds of ampicillin-resistant colonies were obtained. DNA analysis showed they all contained sequences derived from the pHelp1 plasmid. Cosmid DNA isolated from *E*. *coli* colonies was then retransformed into 13PAP2. Two DNA samples gave rise to transformants which showed both growth on acetamide containing medium and increased protease activity. DNA from one of the cosmids, ACR1, was then digested with several restriction enzymes. The resulting fragments were then co-transformed with pAopyrGcosArp1 into strain 13PAP2. EcoRI, PstI, BamHI and KpnI digestion of ACR1 gave rise to transformants capable of growth on acetamide and high protease activity, whereas SalI and HindIII digests did not. Because EcoRI digestion gave the simplest pattern, separate EcoRI fragments were gel-isolated and with pAopyrGcosArp1 used to co-transform 13PAP2. Only one fragment, a 15 kb EcoRI fragment, gave rise to transformants capable of growth on acetamide-containing medium. This fragment was subcloned in pBluescript II SK in order to subclone *prtT* from the cosmid. Since the insert of this clone was still rather large, separate PstI bands were gel isolated and each was co-transformed with pAopyrGcosArp1 into 13PAP2. Only one band, a 2.5 kb PstI fragment, gave rise to transformants that could grow on acetamide-containing medium. This fragment was subcloned in pBlueScript II SK. Four subclones, ClE 0.7, ClE 1.8, NcE 1.1 and NcE 1.4, were constructed from this plasmid based on the restriction map. In addition, a 6.5 kb SstI/EcoRI fragment encompassing the 2.5 kb PstI fragment was subcloned, resulting in pEES1 (shown in Fig. 3).

Southern blot analysis of genomic DNA from AB4.1 showed the presence of only one copy of *prtT.*

### Example 2: Sequencing of the prtT gene and analysis of the sequence

All sequence reactions were prepared using dRhodamine Terminator Cycle Sequencing Kits or BigDye^{™} Terminator Cycle Sequencing Kits from the Perkin-Elmer Corporation (Branchburg NJ, USA). The reactions were run on an ABI PRISM^{®} 377 DNA Sequencer (Perkin-Elmer Corporation) following the manufacturer's instructions.

The *prtT* gene was sequenced from the genomic clones ClE 0.7, ClE 1.8, NcE 1.1, NcE 1.4 and pEES1. The sequence specific primers used are listed below:
122958: CGA TCG ATG ACT GCC TGT
122956: AGA GAC ACA TAG TGC CTT
122959: GCT TAT AGT CGA TAG CGC
122960: CCT CTC TCC AGC GAT GGT
122962: ATG GAA TAC ATA CTG CTT
122961: ATG AAA CCC ACT GTA GCT
122963: TGC TCG ATA AGC GGG TCC
122964: AAT CTT ATG GAC CCG CTT
124289: CCC CGG GAA ACA AGA ACA GG
124290: GTT GGC GGA CCT TGA CTA TG
125112: ACA GCT ACA GTG GGT TTC ATC T
125111: AGT CAA CGG GGG AAG TCT C
128330: CTA GCA GCG TAT CGG TCA GC
130887: CTT GGA AAA GAA ACG ATA G
130888: AAC GTA CGC TTT CCT CCT T
134135: GGG TCC GTC CAG TCC GTT CTT

-48 reverse: AGC GGA TAA CAA TTT CAC ACA GGA
-40 universal: GTT TTC CCA GTC ACG AC

A mutant allele of the gene was obtained by PCR amplification of genomic DNA isolated from the mutant strain AB1.13 using the following primers:
PstI: TC ATC CCT GGT GTT ACT GC
PstII: C ATG GAT TGG CTG GCC G

The complete DNA sequence of the *prtT* gene is shown in SEQ ID NO:1. The sequence of the PCR fragment of the mutant allele is shown in SEQ ID NO:4.

Analysing the DNA sequence SEQ ID NO:1 using the computer software Netgene 2 (S.M. Hebsgaard, P.G. Korning, N. Tolstrup, J. Engelbrecht, P. Rouze, S. Brunak (1996. Nucleic Acids Research 24: 3439-3452) suggested the existence of 5 exons (see annotations to SEQ ID NO 1).

### Analysis of prtT cDNA

mRNA was purified from total RNA (isolated according to the DNA isolation method described above in Example 1) using a commercial poly(A)⁺ RNA isolation kit (Pharmacia, Uppsala SE) from a culture of *A*. *niger* grown under conditions favourable for protease production (J.P.T.W. Van Den Hombergh, *et al.,* 1997. Eur. J. Biochem. 247:605-613). Double stranded cDNA was prepared using standard procedures and used for PCR reactions with the following primers:
oligo-dT primer: T₂₀N
Prt270n: TACTCTCCAGATTGCCTG
Prt1420r: TGAGATACCACTCAGCAG
prt1350n: TGCACTTCTCTGTCTCTG
Prt2365r: GACTTCTGGCATCAGTTG
prt2320n: CTCATGGATGGCATGATC

A PCR reaction with the primers Prt270n and Prt1420r produced a fragment of approximately 1.0 kb. The fragment was cloned into a pGEM-T vector (Promega Corp., Madison WI, USA), and the insert in the resulting plasmid was sequenced using the primers 122958, 122960, -40 universal and -48 reverse. The result confirmed the presence of two introns in this part of the gene.

A second PCR reaction with the primers Prt1350n and Prt2365r produced a fragment of approximately 0.9 kb. This fragment was also cloned in a pGEM-T vector, and the insert in the resulting plasmid was sequenced using the primers 124289, 124290, -40 universal and -48 reverse. The result confirmed the presence of a single intron in this part of the gene.

Another PCR reaction with the oligo-dT primer and primer Prt2320n produced a fragment of approximately 350 bp. This fragment was also cloned in a pGEM-T vector. Sequencing of the insert using primers -40 universal and -48 reverse showed that the fragment contained the 3' part of *prtT* and confirmed the presence of another intron.

The deduced protein sequence of the translated *prtT* gene is shown in SEQ ID NO:2. The deduced protein sequence of the translated mutant allele *prt13* is shown in SEQ ID NO:5. A comparison of SEQ ID NO:2 and SEQ ID NO:5 indicates that the only difference between the two is in position 112 where the leucine residue in the translated *prtT* gene is replaced by proline in the translated *prt13* gene.

Analysis of the deduced PrtT protein sequence reveals the presence of a Zinc(II)2Cys6 binuclear cluster DNA binding motif (SEQ ID NO:2, residues 47-81). This motif defines the GAL4 class of fungal transcriptional activators (Reece, M. J., and Ptashne, M. 1993. Science 261: 909-911). The presence of the motif in the *prtT* gene strongly indicates that prtT is a transcriptional activator.

### EXAMPLE 3: Disruption of the prtT gene in a wild-type A. niger strain

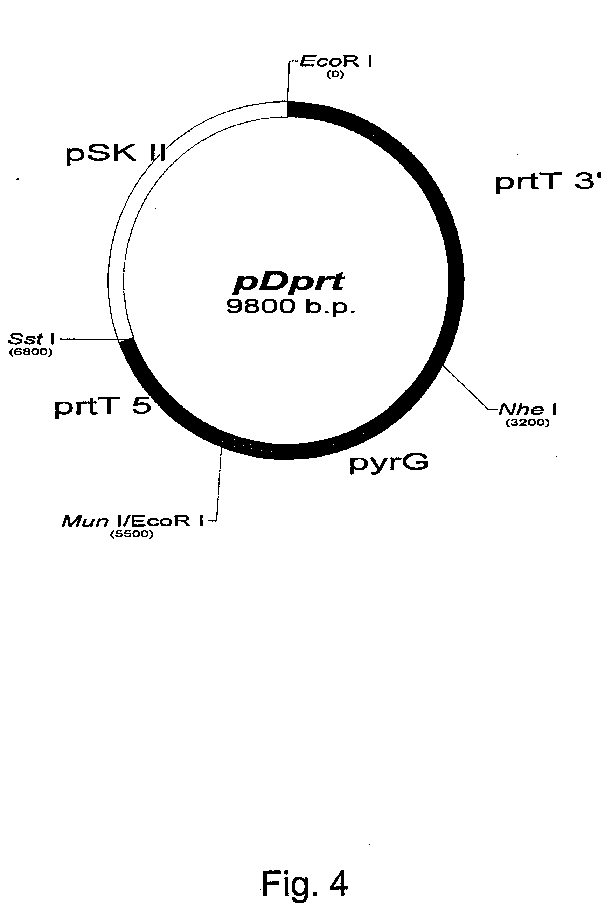

A plasmid was constructed in which the upstream and downstream sequences of the *prtT* gene are separated by the *A*. *oryzae pyrG* gene. Plasmid pEES1 was digested with MunI and NheI which removed a 2.1 kb fragment containing most of the coding sequence of *prtT.* A 2.3 kb EcoRI/NheI fragment from pAO4-13 containing the *A*. *oryzae pyrG* gene was cloned in the MunI and NheI sites of pEES1. The resulting plasmid, shown in Fig. 4, was named pDprt. This construct was then used to transform *A. niger* strain AB4.1 to uridine prototrophy. About 150 uridine prototrophic transformants were then analyzed for protease activity on skim milk containing plates. Five of these did not make a halo on these plates indicating that protease activity was very low. Comparison of strains with a disrupted *prtT* gene and the mutant AB1.13 strain did not show any differences in protease activity or phenotype.

### EXAMPLE 4: Overexpression of PrtT

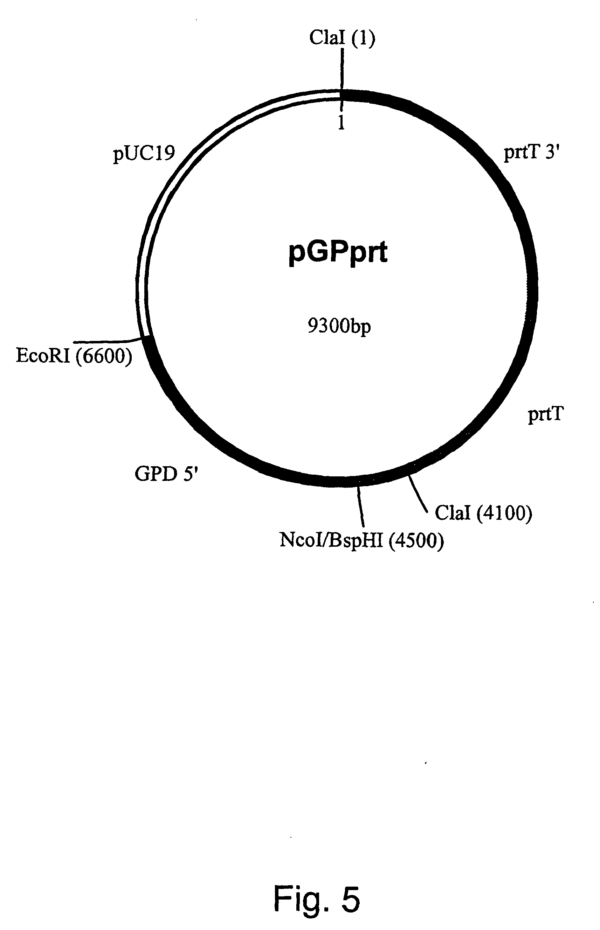

A plasmid, pGPprt, (Figure 5) containing the coding region and 3' noncoding sequences of *prtT* fused to the promoter of the *A*. *niger gpd* gene was constructed. The *gpd* gene codes for glyceraldehyde-3-phosphate dehydrogenase, a constitutively expressed enzyme involved in primary metabolism. The promoter used was a fragment upstream of the coding region.
The plasmid is transformed into A. niger AB4.1 by cotransformation with the pyrG selection plasmid pAO4-13. Transformants with increased prtT transcription as determined by Southern blot analysis is analysed for increased protease expression.

### Deposit of Biological Materials

The following biological material has been deposited under the terms of the Budapest Treaty with the Deutsche Sammlung von Microorganismen und Zellkulturen GmbH, Mascheroder Weg 1b, D-38124 Braunschweig, Germany and given the following accession number:

| Deposit | Accession Number | Date of Deposit |
|---|---|---|
| *Escherichia coli,* pEES | DSM 12294 | 1998-07-14 |
| *Aspergillus niger* 13PAP2 | DSM 12298 | 1998-07-14 |

The strain has been deposited under conditions that assure that access to the culture will be available during the pendency of this patent application. The deposit represents a substantially pure culture of the deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims.

## Claims

1. An isolated nucleic acid sequence encoding a polypeptide having transcriptional activation activity on protease promoters, selected from the group consisting of:
(a) a nucleic acid sequence having at least 70% identity with the nucleic acid sequence of SEQ ID NO:1;
(b) a nucleic acid sequence encoding a polypeptide having an amino acid sequence which has at least 50% identity with the amino acid sequence of SEQ ID NO:2;
(c) a nucleic acid sequence which hybridizes under medium stringency conditions with (i) the nucleic acid sequence of SEQ ID NO:1, or (ii) its complementary strand, wherein the medium stringency conditions are defined by prehybridization and hybridization at 42°C in 5x SSPE, 0.3% SDS, 200 µg/ml sheared and denatured salmon sperm DNA, and 35% formamide, and wash conditions are defined by 50°C for 30 minutes in 2X SSC, 0.2% SDS; and
(d) a subsequence of (a), (b), or (c), wherein the subsequence encodes a polypeptide with the amino acid sequence of SEQ ID NO:3.

2. The nucleic acid sequence of claim 1, wherein the nucleic acid sequence is obtained from a fungal cell or a yeast cell.

3. A nucleic acid construct comprising the nucleic acid sequence of any of claims 1 or 2 operably linked to one or more control sequences which direct the production of the polypeptide in a suitable expression host.

4. An expression vector comprising the nucleic acid construct of claim 3, a promoter, and transcriptional and translational stop signals.

5. A host cell comprising the nucleic acid construct of claim 3 or the expression vector of claim 4.

6. An isolated polypeptide selected from the group consisting of:
(a) a polypeptide which is encoded in a nucleic acid sequence which hybridizes under medium stringency conditions with (i) the nucleic acid sequence of SEQ ID NO:1; (ii) its complementary strand, or (iii) a subsequence of SEQ ID NO:1 which encodes a polypeptide fragment which has transcriptional activation activity, wherein the medium stringency conditions are defined by prehybridization and hybridization at 42°C in 5X SSPE, 0.3% SDS, 200 µg/ml sheared and denatured salmon sperm DNA, and 35% formamide, and wash conditions are defined at 50°C for 30 minutes in 2X SSC, 0.2% SDS;
(b) a polypeptide having an amino acid sequence which has at least 50% identity with the amino acid sequence of SEQ ID NO:2; and
(c) a polypeptide comprising the amino acid sequence of SEQ ID NO:3, or an allelic variant thereof.

7. A method for producing the polypeptide of claim 6 comprising (a) cultivating the host cell of claim 5 under conditions conducive for production of the polypeptide; and (b) recovering the polypeptide.

## Patentansprüche

1. Isolierte Nukleinsäuresequenz, welche ein Polypeptid kodiert, das Transkriptionsaktivierungsaktivität gegenüber Proteasepromotoren aufweist, ausgewählt aus der Gruppe bestehend aus:
(a) einer Nukleinsäuresequenz, welche mindestens 70 % Identität mit der Nukleinsäuresequenz aus SEQ ID NR: 1 aufweist;
(b) einer Nukleinsäuresequenz, welche ein Polypeptid kodiert, das eine Aminosäuresequenz aufweist, welche mindestens 50 % Identität mit der Aminosäuresequenz aus SEQ ID NR: 2 aufweist;
(c) einer Nukleinsäuresequenz, welche unter mittleren Stringenzbedingungen mit
(i) der Nukleinsäuresequenz aus SEQ ID NR: 1 oder
(ii) ihrem komplementären Strang
hybridisiert, wobei die mittleren Stringenzbedingungen durch die Prähybridisierung und Hybridisierung bei 42 °C in 5 x SSPE, 0,3 % SDS, 200 µg/ml gescherter und denaturierter Lachssperma-DNA und 35% Formamid definiert sind, und wobei die Waschbedingungen bei 50 °C für 30 Minuten in 2 x SSC, 0,2 % SDS definiert sind; und
(d) einer Untersequenz von (a), (b) oder (c), wobei die Untersequenz ein Polypeptid mit der Aminosäuresequenz aus SEQ ID NR: 3 kodiert.

2. Nukleinsäuresequenz nach Anspruch 1, wobei die Nukleinsäuresequenz von einer Pilzzelle oder einer Hefezelle erhalten wird.

3. Nukleinsäurekonstrukt, welches die Nukleinsäuresequenz nach einem beliebigen der Ansprüche 1 oder 2 umfasst, die funktionsfähig mit einer oder mehreren Kontrollsequenz(en) verknüpft ist/sind, welche die Herstellung des Polypeptids in einem geeigneten Expressionswirt steuert/steuern.

4. Expressionsvektor, welcher das Nukleinsäurekonstrukt nach Anspruch 3, einen Promotor sowie Transkriptions- und Translationsstopsignale umfasst.

5. Wirtszelle, welche das Nukleinsäurekonstrukt nach Anspruch 3 oder den Expressionsvektor nach Anspruch 4 umfasst.

6. Isoliertes Polypeptid, ausgewählt aus der Gruppe bestehend aus:
(a) einem Polypeptid, welches durch eine Nukleinsäuresequenz kodiert wird, welche unter mittleren Stringenzbedingungen mit
(i) der Nukleinsäuresequenz aus SEQ ID NR: 1;
(ii) ihrem komplementären Strang oder
(iii) einer Untersequenz aus SEQ ID NR: 1, welche ein Polypeptidfragment kodiert, das Transkriptionsaktivierungsaktivität aufweist,
hybridisiert, wobei die mittleren Stringenzbedingungen durch die Prähybridisierung und Hybridisierung bei 42 °C in 5 x SSPE, 0,3 % SDS, 200 µg/ml gescherter und denaturierter Lachssperma-DNA und 35% Formamid definiert sind, und wobei die Waschbedingungen bei 50 °C für 30 Minuten in 2 x SSC, 0,2 % SDS definiert sind;
(b) einem Polypeptid, das eine Aminosäuresequenz aufweist, welche mindestens 50 % Identität mit der Aminosäuresequenz aus SEQ ID NR: 2 aufweist; und
(c) einem Polypeptid, welches die Aminosäuresequenz aus SEQ ID NR: 3 oder eine allele Variante hiervon umfasst.

7. Verfahren zur Herstellung des Polypeptids nach Anspruch 6, umfassend
(a) Kultivieren der Wirtszelle nach Anspruch 5 unter Bedingungen, die der Herstellung des Polypeptids zuträglich sind; und
(b) Gewinnen des Polypeptids.

## Revendications

1. Séquence d'acide nucléique isolée codant un polypeptide ayant une activité d'activation transcriptionnelle sur des promoteurs de protéases, choisie dans le groupe constitué de :
(a) une séquence d'acide nucléique ayant au moins 70 % d'identité avec la séquence d'acide nucléique de SEQ ID NO:1;
(b) une séquence d'acide nucléique codant un polypeptide ayant une séquence en acides aminés qui a au moins 50 % d'identité avec la séquence en acides aminés de SEQ ID NO :2;
(c) une séquence d'acide nucléique qui s'hybride dans des conditions de stringence moyenne avec (i) la séquence d'acide nucléique de SEQ ID NO :1, ou (ii) son brin complémentaire, les conditions de stringence moyenne étant définies par une préhybridation et une hybridation à 42°C dans 5X SSPE, 0,3 % de SDS, 200 µg/ml d'ADN de sperme de saumon fragmenté et dénaturé et 35 % de formamide, et les conditions de lavage étant définies par 50°C pendant 30 minutes dans 2X SSC, 0,2 % SDS ; et
(d) une sous-séquence de (a), (b) ou (c), la sous-séquence codant un polypeptide ayant la séquence en acides aminés de SEQ ID NO :3.

2. Séquence d'acide nucléique selon la revendication 1, dans laquelle la séquence d'acide nucléique est obtenue à partir d'une cellule de champignon ou une cellule de levure.

3. Construction d'acide nucléique comprenant la séquence d'acide nucléique selon l'une quelconque des revendications 1 ou 2, liée de manière opérationnelle à une ou plusieurs séquences de contrôle qui dirigent la production du polypeptide dans un hôte d'expression adapté.

4. Vecteur d'expression comprenant la construction d'acide nucléique selon la revendication 3, un promoteur et des signaux d'arrêt de la transcription et de la traduction.

5. Cellule hôte comprenant la construction d'acide nucléique selon la revendication 3 ou le vecteur d'expression selon la revendication 4.

6. Polypeptide isolé choisi dans le groupe constitué de :
(a) un polypeptide qui est codé dans une séquence d'acide nucléique qui s'hybride dans des conditions de stringence moyenne avec (i) la séquence d'acide nucléique de SEQ ID NO :1 ; (ii) son brin complémentaire ; ou (iii) une sous-séquence de SEQ ID NO :1 qui code un fragment polypeptidique qui a une activité d'activation transcriptionnelle, les conditions de stringence moyenne étant définies par une préhybridation et une hybridation à 42°C dans 5X SSPE, 0,3 % SDS, 200 µg/ml d'ADN de sperme de saumon fragmenté et dénaturé, et 35 % de formamide, et les conditions de lavage étant définies à 50°C pendant 30 minutes dans 2X SSC, 0,2% SDS ;
(b) un polypeptide ayant une séquence en acides aminés qui a au moins 50 % d'identité avec la séquence en acides aminés de SEQ ID NO :2 ; et
(c) un polypeptide comprenant la séquence en acides aminés de SEQ ID NO :3 ou un variant allélique de celui-ci.

7. Méthode pour produire le polypeptide de la revendication 6, comprenant (a) la culture de la cellule hôte de la revendication 5 dans des conditions permettant la production du polypeptide ; et (b) la récupération du polypeptide.


### SEQUENCE LISTING

(1) GENERAL INFORMATION:
   (i) APPLICANT:
      (A) NAME: Novo Nordisk A/S
      (B) STREET: Novo Alle
      (C) CITY: Bagsvaerd
      (E) COUNTRY: Denmark
      (F) POSTAL CODE (ZIP): 2880
      (G) TELEPHONE: +45 4444 8888
   (ii) TITLE OF INVENTION: TITLE
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
(2) INFORMATION FOR SEQ ID NO: 1:
   (i) SEQUENCE CHARACTERISTICS:
      (A) LENGTH: 4098 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (B) STRAIN: DSM 12294
   (ix) FEATURE:
      (A) NAME/KEY: exon
      (B) LOCATION:978..1204
   (ix) FEATURE:
      (A) NAME/KEY: exon
      (B) LOCATION:1317..1718
   (ix) FEATURE:
      (A) NAME/KEY: exon
      (B) LOCATION:1777..2202
   (ix) FEATURE:
      (A) NAME/KEY: exon
      (B) LOCATION:2253..3116
   (ix) FEATURE:
      (A) NAME/KEY: exon
      (B) LOCATION:3173..3248
   (xi) SEQUENCE DESCRIPTION: SEQ ID NO: 1:
   TTGGTGCTGG AAAGCCCATT TAAGGGATCT TATAAGGTAA TTGCCAATGT TCAGTCGCCT      60
   ATGGTCTTTG TCGAGAGAAA CTCTTTCTCG TTAAGATCTA CATGATCGCT TTTGATTTTC     120
   TCTGGGTTCA CGCGGTACTT TCTCCCCGTC AATCCCCAAC CGCTGTTGTG CCTGACCATC     180
   AATGTGGAAC GGATAAGGGG ACAAGAGAAA TTGAAGGAGC GATCATAAAA AGCTAATTTT     240
   GGTTTATTAT TTTTTTTTCT TATAAAACTC AAAAAAGAAA ACGAAAACGA AAAAGGAAAA     300
   AAGAAAAGGT AAAATGGAAA AAGAAAGGCG GTCATCACTT CCAATAACCA TCAGCCAAAG     360
   ATACAGACGA GTTACTGACC TTCTTATCCT GGACTTCCGC CCGATCCATA TCTTCATGAT     420
   AAGCAGGGAA CCGAACAAAT CAACGCCAAC TTCAGCGGCA GTTCCTCACT AATTTCCCAC     480
   TTCCCACCGG CGTCATTTTG GTCCCAACCC CCTCCCTGGA AGCAGCGGGA TTTAGTTACG     540
   ATCCGGTTTA CATCGGAGAC TCGGAAAATA CCATAGCGCA TGCCAATCAA AACCCCTCCC     600
   AGGGTGACTG GCCAGTATCA CGACCCATTG TTTCTATCTT TCTAGAAGAC CTGCAGGGAC     660
   ATGGATTGGC TGGCCGCCGT GCTGCCGTCC ATTAGCGTCT ACCCCAGGTC AAGAACGGAC     720
   TGGACGGACC CATAACCAAT CTAACCAAAG CCAATTTCGT CAATTCCCAG CTGGCGAGCA     780
   CAATCCCATT CCCAGGGTTG GCCGCCAACT GTTAAAAGGC ACTATGTGTC TCTCCACCTG     840
   CCCGCCCCCC TCGATGGCCT GCGCGTAATA ACTATTCTAC TGCTTTTTGC CTCTTACTTG     900
   CCTCATTATT AGTATTTTAC TCTACTCTCC AGATTGCCTG CCAGCAATTG GTCCAAAGTG     960
   GACTTTGTTT GATGACATGA CTCGAACCGT GGACGAGATC AAATACGAAA CGCCTTCTTC    1020
   ATGGGAGCAC AAGAGCTTGG ACGTTGCCGA GGATGGCAGG CGACTAGCTC CCCATTCCGA    1080
   CACTGCTCGT CCGAAAGGCC GCATACGACG ATCGATGACT GCCTGTCACA CATGTCGGAA    1140
   GCTTAAAACT AGATGTGATC TAGATCCGCG CGGTCATGCG TGCCGTCGCT GTCTATCTCT    1200
   AAGGTCAGAG GCACTACCTA CCTGCCAGTT GAAGCTTTGT CCTTCTGAAC GCGACATGAT    1260
   ACTAGTCGTG GAATATAACT GTCCCAACTT TGCTGACAGT CCACAATATC TTTAGAATCG    1320
   ATTGTAAGCT GCCTGAAACG ACCGACCGCT TCCAAGACAG TGCTGCGATG TGGCCAGACG    1380
   CCACCTCGGC AATTCCCTCC ATCGAGGAGC GCCTCACCTC CCTAGAAAGA TGCATGAGGG    1440
   AGATGACGGG CATGATGCGA CAGATGCTAG ATCACTCCCC AGGTTTCGCA AATGCCTCGG    1500
   TTCCGCATTT GACCAAAAGC ATCATCACGG ATGAAACCGC CTCGATGGAG GGAAGCCCGT    1560
   CGTCCCCCTT CCTGCCTAAG CCCGTTCGCC TCATTCAGGA CCTCCAGTCC GACTTCTTCG    1620
   GAGAAGCAGA GACTTCCCCC GTTGACTCCC CTCTCTCCAG CGATGGTAAC GCCAAGGGCG    1680
   CTATCGACTC TAAGCTATCC CTCAAATTGT TGCAAACGTA TGGGTATACC TGATTGACAA    1740
   TTACCAAAAA GCTGCTAATC CTTGGCGCAA ATCAGGTTTG TCGATCACTT TGGCGCTTGC   1800
   GTTTCCATTT ACAATCTCTC CGACATCCAC AACGACATGA AAGCCCCCGA CTCTTTACTG   1860
   TATAATACTG CATGCCTTCT AGCTTCACGC TATGTACCGG GGATACCGAC ATCTACCGTG   1920
   CATGCTATAT ACCTTCAAGT GCGACATGCA GTAGTCAATA TTTTGTGGGA AAAACCACCC   1980
   CTGAAGTATG AGACCCTCCA AGCACTTGCA CTTCTCTGTC TCTGGCCAGC AACCGCCCAG   2040
   AAAGAGCCAC CCATGGACAG CTGGCTGCTG AGTGGTATCT CAATTAACCA TGCAATTATC   2100
   GCGCTCGATT TCCTAAACTA TGCGCCCTCG GAAGTCATGG TGGACAATGA AACGGCTGCG   2160
   CAGCTGCGGC TATGGAATAC ATATTGCTTG ACACAGCTAC AGTGGGTTTC ATCTAAGATC   2220
   TCCCGTCCAG AAGATAGCTA ACAAGCTTTA GTTTTGCGGT CGGGAATGCG CGTCCTTTCC   2280
   ATATCCAGCA AAGATACCTT GACCACTGCC CACGGATACT GGAGCACCCA GCAGCAACTC   2340
   TGGAGGACGC AAGGGTTGTA GCAGAAATAC AGTTGTATTT GATGACATTG CGGCTCCAGA   2400
   GCAATAGCAG TCGAATGCGG TTGGCGGACC TTGACTATGA GGAAATAGAG CGATGGAAGA   2460
   GGGAGTGGGC TCACCTTTTC TGTAAGAAGC CTGTTCTTGT TTCCCGGGGA CTACCACTGA   2520
   CGAGAGCAAC AGCTGGGGAA AGTTCCACAT TGGAGCTGAG CCTTTGGTTC TGCCAGACAC   2580
   TCCTTCACCG CACAGCAATG AGGCTTCAGC CCAGATCCGA CAGGCTCGCA TCTGAGGTTC   2640
   TGCAAACCTC ACGTCTGATA ATATCGCGGT TCCTCCAGAT CCGGTACTCT ACCGCATTAA   2700
   GCCTTGTCGA CCAAGTCTAT TTCATTGTCG GCTACGCTGC ACTGAATCTG TGCGATTTCA   2760
   ATCTTATGGA CCCGCTTATC GAGCAAGTGC AGATGTTCCT GCTGCATCTC TCCCCGAACG   2820
   AAGACCACAT CGCCTACCGG TTTTCGTGCA TGGTCGCCGA GTTCAAGCGG CGATGTGGCA   2880
   GTGCGGAATG CAATGACCCA TCATCCACTG TCAAGGGGTC TCCGTTATCA TCCTACGGCG   2940
   ACAGTCGTAA GATGAGCATG GGGCAAGCAC CGTTCATGCC ACCGCTCATG GATGGCATGA   3000
   TCGAGGGGTA CGGCTTCGAG CAACTGATGC CAGAAGTCAT GCCGAGTTCC TTTCCGGATG   3060
   GGATACTCAA CGGAATGCCT GTGACTGGGC TAGCAGCGTA TCGGTCAGCG ACGCTGTAAG   3120
   TAATCGAGAT CGGGTTGGAA AGGACATGAG TGGGGGTGGT GGTGGTAGTA GCAGTAACAC   3180
   CAGGGATGAT AACCTGCAGC GGTGGTTTAG TTCCTGCCCA TGGGCTGAAC TAAAACCCCG   3240
   AACCTAGCAT GATGACGTGC AACGAAAGGA TCATAACCAA GGCCAAGTAA ATACTAAAAT   3300
   AAAATAATAT AATTCCACAC GATCCACTAC CACCACCACC ACCGGATCCA TCAGGTTGCC   3360
   TTCCTGCACA GGCCTATTTA GTTAGAGGGC CCGTGCCACG AAACATCACG TAATTGAGCG   3420
   CTTTTGCTTC CTTGCAACTT AAACAACCCC ATAGACACTC TCACATTCAC ATGCCAAACT   3480
   ACTAACTCCT ACTGACCACC AGCTGCAGGA AGCCAGCCAG CCACCATTTC CTAATCGGAT   3540
   ATATCTCCGA AACGTACGCT TTCCTCCTTT GTTCGGACCG TTCCGTGCCT CCGCGGAGAG 3600
   TTGAACGAGT CAGAACACAT TCTTTTCGTT TCTATCGTTT CTTTTCCAAG GCAGCAGAGA 3660
   GACGAACAAG TCAGTGCTTG CTAACTAACT TACCCCTCAG CATTTTAGTA AACTACTATT 3720
   TAGGAAAGAG TAATCATTCA TCGAAGACAA GATGTTTATT TCTCCGATCG ACCAAACAAA 3780
   AACGTTCAGG TAGACTAAGT AGTAGTAGTA GTATGTCTTT GACCCCTTTA CTCCACTATC 3840
   CGTTGACTGC ACATAGTAGT AAGTAACTAT CTAACCAGTT GCCGAGGAGA GGAAAGTGAG 3900
   TGGGTGGGAG CCGGAGGATG CCGCCGAGAA TTATTAAGTC GATCATTGCT AGTTAGTTAT 3960
   CTTTTCATGA TGAGGAGAGG AAGGAGAGGG GGGACGGGAT TAGAGAAATA AACTTTTCTC 4020
   TCCAATTAAT TATCTGGATT AATTAAAACT TGGAGAGGAG GGTAGGGGAG TTGGGTATTG 4080
   GTATGTTGCT GTGAATGT 4098
(2) INFORMATION FOR SEQ ID NO: 2:
   (i) SEQUENCE CHARACTERISTICS:
      (A) LENGTH: 666 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 2:
   Met Thr Arg Thr Val Asp Glu Ile Lys Tyr Glu Thr Pro Ser Ser Trp
   1               5                   10                  15
   Glu His Lys Ser Leu Asp Val Ala Glu Asp Gly Arg Arg Leu Ala Pro
               20                  25                  30
   His Ser Asp Thr Ala Arg Pro Lys Gly Arg Ile Arg Arg Ser Met Thr
           35                  40                  45
   Ala Cys His Thr Cys Arg Lys Leu Lys Thr Arg Cys Asp Leu Asp Pro
       50                  55                  60
   Arg Gly His Ala Cys Arg Arg Cys Leu Ser Leu Arg Ile Asp Cys Lys
   65                  70                  75                  80
   Leu Pro Glu Thr Thr Asp Arg Phe Gln Asp Ser Ala Ala Met Trp Pro
                   85                  90                  95
   Asp Ala Thr Ser Ala Ile Pro Ser Ile Glu Glu Arg Leu Thr Ser Leu
               100                 105                 110
   Glu Arg Cys Met Arg Glu Met Thr Gly Met Met Arg Gln Met Leu Asp
           115                 120                 125
   His Ser Pro Gly Phe Ala Asn Ala Ser Val Pro His Leu Thr Lys Ser
       130                 135                 140
   Ile Ile Thr Asp Glu Thr Ala Ser Met Glu Gly Ser Pro Ser Ser Pro
   145                 150                 155                 160
   Phe Leu Pro Lys Pro Val Arg Leu Ile Gln Asp Leu Gln Ser Asp Phe
                   165                 170                 175
   Phe Gly Glu Ala Glu Thr Ser Pro Val Asp Ser Pro Leu Ser Ser Asp
               180                 185                 190
   Gly Asn Ala Lys Gly Ala Ile Asp Ser Lys Leu Ser Leu Lys Leu Leu
           195                 200                 205
   Gln Thr Phe Val Asp His Phe Gly Ala Cys Val Ser Ile Tyr Asn Leu
       210                 215                 220
   Ser Asp Ile His Asn Asp Met Lys Ala Pro Asp Ser Leu Leu Tyr Asn
   225                 230                 235                 240
   Thr Ala Cys Leu Leu Ala Ser Arg Tyr Val Pro Gly Ile Pro Thr Ser
                   245                 250                 255
   Thr Val His Ala Ile Tyr Leu Gln Val Arg His Ala Val Val Asn Ile
               260                 265                 270
   Leu Trp Glu Lys Pro Pro Leu Lys Tyr Glu Thr Leu Gln Ala Leu Ala
           275                 280                 285
   Leu Leu Cys Leu Trp Pro Ala Thr Ala Gln Lys Glu Pro Pro Met Asp
       290                 295                 300
   Ser Trp Leu Leu Ser Gly Ile Ser Ile Asn His Ala Ile Ile Ala Leu
   305                 310                 315                 320
   Asp Phe Leu Asn Tyr Ala Pro Ser Glu Val Met Val Asp Asn Glu Thr
                   325                 330                 335
   Ala Ala Gln Leu Arg Leu Trp Asn Thr Tyr Cys Leu Thr Gln Leu His
               340                 345                 350
   Phe Ala Val Gly Asn Ala Arg Pro Phe His Ile Gln Gln Arg Tyr Leu
           355                 360                 365
   Asp His Cys Pro Arg Ile Leu Glu His Pro Ala Ala Thr Leu Glu Asp
       370                 375                 380
   Ala Arg Val Val Ala Glu Ile Gln Leu Tyr Leu Met Thr Leu Arg Leu
   385                 390                 395                 400
   Gln Ser Asn Ser Ser Arg Met Arg Leu Ala Asp Leu Asp Tyr Glu Glu
                   405                 410                 415
   Ile Glu Arg Trp Lys Arg Glu Trp Ala His Leu Phe Cys Lys Lys Pro
               420                 425                 430
   Val Leu Val Ser Arg Gly Leu Pro Leu Thr Arg Ala Thr Ala Gly Glu
           435                 440                 445
   Ser Ser Thr Leu Glu Leu Ser Leu Trp Phe Cys Gln Thr Leu Leu His
       450                 455                 460
   Arg Thr Ala Met Arg Leu Gln Pro Arg Ser Asp Arg Leu Ala Ser Glu
   465                 470                 475                 480
   Val Leu Gln Thr Ser Arg Leu Ile Ile Ser Arg Phe Leu Gln Ile Arg
                   485                 490                 495
   Tyr Ser Thr Ala Leu Ser Leu Val Asp Gln Val Tyr Phe Ile Val Gly
               500                 505                 510
   Tyr Ala Ala Leu Asn Leu Cys Asp Phe Asn Leu Met Asp Pro Leu Ile
           515                 520                 525
   Glu Gln Val Gln Met Phe Leu Leu His Leu Ser Pro Asn Glu Asp His
       530                 535                 540
   Ile Ala Tyr Arg Phe Ser Cys Met Val Ala Glu Phe Lys Arg Arg Cys
   545                 550                 555                 560
   Gly Ser Ala Glu Cys Asn Asp Pro Ser Ser Thr Val Lys Gly Ser Pro
                   565                 570                 575
   Leu Ser Ser Tyr Gly Asp Ser Arg Lys Met Ser Met Gly Gln Ala Pro
               580                 585                 590
   Phe Met Pro Pro Leu Met Asp Gly Met Ile Glu Gly Tyr Gly Phe Glu
           595                 600                 605
   Gln Leu Met Pro Glu Val Met Pro Ser Ser Phe Pro Asp Gly Ile Leu
       610                 615                 620
   Asn Gly Met Pro Val Thr Gly Leu Ala Ala Tyr Arg Ser Ala Thr Leu
   625                 630                 635                 640
   Ser Ser Asn Thr Arg Asp Asp Asn Leu Gln Arg Trp Phe Ser Ser Cys
                   645                 650                 655
   Pro Trp Ala Glu Leu Lys Pro Arg Thr Pro
               660                 665
(2) INFORMATION FOR SEQ ID NO: 3:
   (i) SEQUENCE CHARACTERISTICS:
      (A) LENGTH: 35 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 3:
   Met Thr Ala Cys His Thr Cys Arg Lys Leu Lys Thr Arg Cys Asp Leu
   1               5                   10                  15
   Asp Pro Arg Gly His Ala Cys Arg Arg Cys Leu Ser Leu Arg Ile Asp
               20                  25                  30
   Cys Lys Leu
           35
(2) INFORMATION FOR SEQ ID NO: 4:
   (i) SEQUENCE CHARACTERISTICS:
      (A) LENGTH: 2542 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PCR Fragment"
   (vi) ORIGINAL SOURCE:
      (B) STRAIN: prt13 allele
   (xi) SEQUENCE DESCRIPTION: SEQ ID NO: 4:
   GACATGGATT GGCTGGCCGC CGTGCTGCCG TCCATTAGCG TCTACCCCAG GTCAAGAACG      60
   GACTGGACGG ACCCATAACC AATCTAACCA AAGCCAATTT CGTCAATTCC CAGCTGGCGA     120
   GCACAATCCC ATTCCCAGGG TTGGCCGCCA ACTGTTAAAA GGCACTATGT GTCTCTCCAC     180
   CTGCCCGCCC CCCTCGATGG CCTGCGCGTA ATAACTATTC TACTGCTTTT TGCCTCTTAC     240
   TTGCCTCATT ATTAGTATTT TACTCTACTC TCCAGATTGC CTGCCAGCAA TTGGTCCAAA     300
   GTGGACTTTG TTTGATGACA TGACTCGAAC CGTGGACGAG ATCAAATACG AAACGCCTTC     360
   TTCATGGGAG CACAAGAGCT TGGACGTTGC CGAGGATGGC AGGCGACTAG CTCCCCATTC     420
   CGACACTGCT CGTCCGAAAG GCCGCATACG ACGATCGATG ACTGCCTGTC ACACATGTCG     480
   GAAGCTTAAA ACTAGATGTG ATCTAGATCC GCGCGGTCAT GCGTGCCGTC GCTGTCTATC     540
   TCTAAGGTCA GAGGCACTAC CTACCTGCCA GTTGAAGCTT TGTCCTTCTG AACGCGACAT     600
   GATACTAGTC GTGGAATATA ACTGTCCCAA CTTTGCTGAC AGTCCACAAT ATCTTTAGAA     660
   TCGATTGTAA GCTGCCTGAA ACGACCGACC GCTTCCAAGA CAGTGCTGCG ATGTGGCCAG     720
   ACGCCACCTC GGCAATTCCC TCCATCGAGG AGCGCCTCAC CTCCCTAGAA AGATGCATGA     780
   GGGAGATGAC GGGCATGATG CGACAGATGC TAGATCACTC CCCAGGTTTC GCAAATGCCT     840
   CGGTTCCGCA TTTGACCAAA AGCATCATCA CGGATGAAAC CGCCTCGATG GAGGGAAGCC     900
   CGTCGTCCCC CTTCCTGCCT AAGCCCGTTC GCCTCATTCA GGACCTCCAG TCCGACTTCT     960
   TCGGAGAAGC AGAGACTTCC CCCGTTGACT CCCCTCTCTC CAGCGATGGT AACGCCAAGG    1020
   GCGCTATCGA CTCTAAGCTA TCCCTCAAAT TGTTGCAAAC GTATGGGTAT ACCTGATTGA    1080
   CAATTACCAA AAAGCTGCTA ATCCTTGGCG CAAATCAGGT TTGTCGATCA CTTTGGCGCT    1140
   TGCGTTTCCA TTTACAATCT CTCCGACATC CACAACGACA TGAAAGCCCC CGACTCTTTA    1200
   CTGTATAATA CTGCATGCCT TCTAGCTTCA CGCTATGTAC CGGGGATACC GACATCTACC    1260
   GTGCATGCTA TATACCTTCA AGTGCGACAT GCAGTAGTCA ATATTTTGTG GGAAAAACCA    1320
   CCCCTGAAGT ATGAGACCCT CCAAGCACTT GCACTTCTCT GTCTCTGGCC AGCAACCGCC    1380
   CAGAAAGAGC CACCCATGGA CAGCTGGCTG CTGAGTGGTA TCTCAATTAA CCATGCAATT    1440
   ATCGCGCTCG ATTTCCTAAA CTATGCGCCC TCGGAAGTCA TGGTGGACAA TGAAACGGCT    1500
   GCGCAGCTGC GGCTATGGAA TACATATTGC TTGACACAGC TACAGTGGGT TTCATCTAAG    1560
   ATCTCCCGTC CAGAAGATAG CTAACAAGCT TTAGTTTTGC GGTCGGGAAT GCGCGTCCTT    1620
   TCCATATCCA GCAAAGATAC CTTGACCACT GCCCACGGAT ACTGGAGCAC CCAGCAGCAA    1680
   CTCTGGAGGA CGCAAGGGTT GTAGCAGAAA TACAGTTGTA TTTGATGACA TTGCGGCTCC    1740
   AGAGCAATAG CAGTCGAATG CGGTTGGCGG ACCTTGACTA TGAGGAAATA GAGCGATGGA    1800
   AGAGGGAGTG GGCTCACCTT TTCTGTAAGA AGCCTGTTCT TGTTTCCCGG GGACTACCAC    1860
   TGACGAGAGC AACAGCTGGG GAAAGTTCCA CATTGGAGCT GAGCCTTTGG TTCTGCCAGA    1920
   CACTCCTTCA CCGCACAGCA ATGAGGCTTC AGCCCAGATC CGACAGGCTC GCATCTGAGG    1980
   TTCTGCAAAC CTCACGTCTG ATAATATCGC GGTTCCTCCA GATCCGGTAC TCTACCGCAT    2040
   TAAGCCTTGT CGACCAAGTC TATTTCATTG TCGGCTACGC TGCACTGAAT CTGTGCGATT    2100
   TCAATCTTAT GGACCCGCTT ATCGAGCAAG TGCAGATGTT CCTGCTGCAT CTCTCCCCGA    2160
   ACGAAGACCA CATCGCCTAC CGGTTTTCGT GCATGGTCGC CGAGTTCAAG CGGCGATGTG    2220
   GCAGTGCGGA ATGCAATGAC CCATCATCCA CTGTCAAGGG GTCTCCGTTA TCATCCTACG    2280
   GCGACAGTCG TAAGATGAGC ATGGGGCAAG CACCGTTCAT GCCACCGCTC ATGGATGGCA    2340
   TGATCGAGGG GTACGGCTTC GAGCAACTGA TGCCAGAAGT CATGCCGAGT TCCTTTCCGG    2400
   ATGGGATACT CAACGGAATG CCTGTGACTG GGCTAGCAGC GTATCGGTCA GCGACGCTGT    2460
   AAGTAATCGA GATCGGGTTG GAAAGGACAT GAGTGGGGGT GGTGGTGGTA GTAGCAGTAA    2520
   CACCAGGGAT GATAACCTGC AG                                            2542
(2) INFORMATION FOR SEQ ID NO: 5:
   (i) SEQUENCE CHARACTERISTICS:
      (A) LENGTH: 665 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (B) STRAIN: prt13 gene product
   (xi) SEQUENCE DESCRIPTION: SEQ ID NO: 5:
   Met Thr Arg Thr Val Asp Glu Ile Lys Tyr Glu Thr Pro Ser Ser Trp
   1               5                   10                  15
   Glu His Lys Ser Leu Asp Val Ala Glu Asp Gly Arg Arg Leu Ala Pro
               20                  25                  30
   His Ser Asp Thr Ala Arg Pro Lys Gly Arg Ile Arg Arg Ser Met Thr
           35                  40                  45
   Ala Cys His Thr Cys Arg Lys Leu Lys Thr Arg Cys Asp Leu Asp Pro
       50                  55                  60
   Arg Gly His Ala Cys Arg Arg Cys Leu Ser Leu Arg Ile Asp Cys Lys
   65                  70                  75                  80
   Leu Pro Glu Thr Thr Asp Arg Phe Gln Asp Ser Ala Ala Met Trp Pro
                   85                  90                  95
   Asp Ala Thr Ser Ala Ile Pro Ser Ile Glu Glu Arg Leu Thr Ser Pro
               100                 105                 110
   Glu Arg Cys Met Arg Glu Met Thr Gly Met Met Arg Gln Met Leu Asp
           115                 120                 125
   His Ser Pro Gly Phe Ala Asn Ala Ser Val Pro His Leu Thr Lys Ser
       130                 135                 140
   Ile Ile Thr Asp Glu Thr Ala Ser Met Glu Gly Ser Pro Ser Ser Pro
   145                 150                 155                 160
   Phe Leu Pro Lys Pro Val Arg Leu Ile Gln Asp Leu Gln Ser Asp Phe
                   165                 170                 175
   Phe Gly Glu Ala Glu Thr Ser Pro Val Asp Ser Pro Leu Ser Ser Asp
               180                 185                 190
   Gly Asn Ala Lys Gly Ala Ile Asp Ser Lys Leu Ser Leu Lys Leu Leu
           195                 200                 205
   Gln Thr Phe Val Asp His Phe Gly Ala Cys Val Ser Ile Tyr Asn Leu
       210                 215                 220
   Ser Asp Ile His Asn Asp Met Lys Ala Pro Asp Ser Leu Leu Tyr Asn
   225                 230                 235                 240
   Thr Ala Cys Leu Leu Ala Ser Arg Tyr Val Pro Gly Ile Pro Thr Ser
                   245                 250                 255
   Thr Val His Ala Ile Tyr Leu Gln Val Arg His Ala Val Val Asn Ile
               260                 265                 270
   Leu Trp Glu Lys Pro Pro Leu Lys Tyr Glu Thr Leu Gln Ala Leu Ala
           275                 280                 285
   Leu Leu Cys Leu Trp Pro Ala Thr Ala Gln Lys Glu Pro Pro Met Asp
       290                 295                 300
   Ser Trp Leu Leu Ser Gly Ile Ser Ile Asn His Ala Ile Ile Ala Leu
   305                 310                 315                 320
   Asp Phe Leu Asn Tyr Ala Pro Ser Glu Val Met Val Asp Asn Glu Thr
                   325                 330                 335
   Ala Ala Gln Leu Arg Leu Trp Asn Thr Tyr Cys Leu Thr Gln Leu His
               340                 345                 350
   Phe Ala Val Gly Asn Ala Arg Pro Phe His Ile Gln Gln Arg Tyr Leu
           355                 360                 365
   Asp His Cys Pro Arg Ile Leu Glu His Pro Ala Ala Thr Leu Glu Asp
       370                 375                 380
   Ala Arg Val Val Ala Glu Ile Gln Leu Tyr Leu Met Thr Leu Arg Leu
   385                 390                 395                 400
   Gln Ser Asn Ser Ser Arg Met Arg Leu Ala Asp Leu Asp Tyr Glu Glu
                   405                 410                 415
   Ile Glu Arg Trp Lys Arg Glu Trp Ala His Leu Phe Cys Lys Lys Pro
               420                 425                 430
   Val Leu Val Ser Arg Gly Leu Pro Leu Thr Arg Ala Thr Ala Gly Glu
           435                 440                 445
   Ser Ser Thr Leu Glu Leu Ser Leu Trp Phe Cys Gln Thr Leu Leu His
       450                 455                 460
   Arg Thr Ala Met Arg Leu Gln Pro Arg Ser Asp Arg Leu Ala Ser Glu
   465                 470                 475                 480
   Val Leu Gln Thr Ser Arg Leu Ile Ile Ser Arg Phe Leu Gln Ile Arg
                   485                 490                 495
   Tyr Ser Thr Ala Leu Ser Leu Val Asp Gln Val Tyr Phe Ile Val Gly
               500                 505                 510
   Tyr Ala Ala Leu Asn Leu Cys Asp Phe Asn Leu Met Asp Pro Leu Ile
           515                 520                 525
   Glu Gln Val Gln Met Phe Leu Leu His Leu Ser Pro Asn Glu Asp His
       530                 535                 540
   Ile Ala Tyr Arg Phe Ser Cys Met Val Ala Glu Phe Lys Arg Arg Cys
   545                 550                 555                 560
   Gly Ser Ala Glu Cys Asn Asp Pro Ser Ser Thr Val Lys Gly Ser Pro
                           565                 570                 575
   Leu Ser Ser Tyr Gly Asp Ser Arg Lys Met Ser Met Gly Gln Ala Pro
               580                 585                 590
   Phe Met Pro Pro Leu Met Asp Gly Met Ile Glu Gly Tyr Gly Phe Glu
           595                 600                 605
   Gln Leu Met Pro Glu Val Met Pro Ser Ser Phe Pro Asp Gly Ile Leu
       610                 615                 620
   Asn Gly Met Pro Val Thr Gly Leu Ala Ala Tyr Arg Ser Ala Thr Leu
   625                 630                 635                     640
   Ser Ser Asn Thr Arg Asp Asp Asn Leu Gln Arg Trp Phe Ser Ser Cys
                   645                 650                 655
   Pro Trp Ala Glu Leu Lys Pro Arg Thr
               660                 665